# EUROPEAN PATENT SPECIFICATION

(11) **EP 1 611 880 B1**
(45) Date of publication and mention of the grant of the patent: **05.11.2008**
(21) Application number: 05105900.4
(22) Date of filing: 30.06.2005
(51) Int. Cl.: A61K 9/20, A61K 31/485

(54) **Pharmaceutical formulations for the safe administration of drugs used in the treatment of drug addiction**
Pharmazeutische Zusammensetzungen zur sicheren Verabreichung von bei der Behandlung von Drogenabhängigkeit verwendeten Arzneimitteln
Compositions pharmaceutiques pour l'administration sûre des médicaments dans le traitement de la narcomanie

(30) Priority: 30.06.2004 IT MI20041317
(43) Date of publication of application: 04.01.2006
(73) Proprietor: ALTERGON S.A., 6903 Lugano (CH)
(72) Inventor: Mateo Echanagorria, Angel, 20090, SEGRATE (IT); Brambilla, Luigi, 26900, LODI (IT); Iacchetti, Giovanni, 26845, CODOGNO (IT)
(74) Representative: Weisgerber, Stefan

(56) References cited:
- WO-A-95/20947
- WO-A-03/013476
- CA-A- 2 454 050
- GB-A- 1 378 348
- US-A- 2 961 374
- US-A- 4 070 494

## Description

### FIELD OF THE INVENTION

The present invention concerns pharmaceutical formulations for drugs used in the treatment of drug addiction and processes for obtaining the same.

### PRIOR ART

Maintenance treatment is widely known throughout the world as a drug addiction treatment, enabling an opiate dependent person to lead a normal and productive life. Maintenance treatment involves the long term therapeutic administration of measured doses of replacement narcotic drugs (preferably methadone, a synthetic narcotic analgesic with long half life) so as to detoxify the addicted individuals from opiates (often heroin) by stopping or reducing the use of this latter, and in particular by stabilizing drug addicts with the replacement narcotic drug for the entire time necessary for them to re-establish their lives and avoid relapsing into previous drug addition patterns.

With regard to the specific case of methadone maintenance treatment (MMT), by far the most practised maintenance worldwide, its usefulness has been recognised by hundreds of scientific studies; negative health consequences attributable to this maintenance treatment hardly exist, even when continued for more than twenty or thirty years. On the other hand, maintenance treatment requires adherence to a very strict administration regimen, necessitating the distribution of a moderate number of daily doses to be taken by the patients as well as rigid patient compliance with self-administration instructions. This is because, although replacement narcotic drugs used in maintenance treatments (particularly methadone) do not possess intoxicating characteristics when administered by prescribed methods, the abusive self-administration thereof, specifically by intravenous means, results instead in a so-called "flash" i.e. the euphoric effect caused by the high plasma peaks attained within a short period, this being totally counterproductive to the aims of the therapy. The risk therefore exists that the patients will attempt to intravenously self-administer the replacement narcotics offered to them (in particular methadone).

Currently, to reduce the possibility of self-administration to a minimum, the daily doses destined for individual consumption within the scope of the methadone maintenance treatment are distributed in the form of single dose ampoules of syrup containing between 5 mg and 100 mg of methadone hydrochloride in 20 ml of liquid per dose. Said syrups have been developed with the purpose of rendering impossible abusive intravenous self-administration, often achieved by dissolving the tablets or emptying (followed by dissolving) the contents of the formerly used capsules. The syrups in current use have the advantage of being too dilute for abusive intravenous self-administration and can be charged by the manufacturer with large quantities of water-soluble excipients, such as sugars, thus rendering difficult any attempts by the drug addict to separate methadone hydrochloride from the rest of the dilute formulation. Given the large volumes of daily doses, said syrups are also secure from another aspect, in that they are unsuitable for abusive administration to third parties without their knowledge, i.e. in cases where criminal individuals attempt to orally administer methadone preparations, previously procured on the black market, to unknowing persons, for example by adding them to a drink with the illicit intent of drugging their victims.

Whilst the currently used syrups are effective for the aforementioned purposes and have been established on the market for decades as preferred formulations for precisely these reasons, their handling during legitimate distribution is burdensome owing to the large volumes involved. This is because the distribution centres necessary for the maintenance treatment of individuals being cared for - given the unstable state of the patients and the ever present consequent risk of the patients relapsing into drug addiction (and their consequent susceptibility to the undertaking of unlawful acts often associated with their illness) - are guarded and protected with a level of security comparable to that of banks. Consequently, provision for the volumes required for storing doses for local distribution poses the not inconsiderable problem of expense, when considering that the volume involved in legitimate distribution in Italy alone of the formulations provided by a single producer amounts to 20 million doses per year. This signifies a massive committed handling of materials already at the very production stage and the distribution centre supply stage, thus production and supply having in their turn to satisfy the same very high standards of security adopted at the final delivery stage, i.e. safety standards which require effective measures against raids, burglary and theft of transacted narcotics, whether by outsiders or members of staff entrusted with carrying out of the necessary duties.

As a consequence, in view of the difficulties found with conventional syrups, and in particular their onerous legitimate distribution, often a burden on public expenditure, the need exists to provide new safe pharmaceutical compositions for drugs used in the treatment of drug addiction (so called replacement narcotics), in particular methadone and/or its salts, more particularly its hydrochloride, as well as procedures for obtaining these formulations. In particular the requirement exists for new formulations that are hard to abuse, i.e. difficult to tamper with in such a way as to be able to be self-administered intravenously.

Advantageously, said new formulations should also be hard to administer orally to unknowing third parties, who are not drug dependent, by criminal individuals who intend to exploit narcotic drug effects in order to commit further illicit acts to the detriment of unknowing third parties. On the other hand, the new formulations must be easier to handle than those used heretofore, thus facilitating their legitimate distribution within the scope of the therapy.

Compositions comprising a drug such as methadone and a gelling agent to prevent abuse are disclosed in WO 03/013476 A, US 4,070,494 A, GB 1 378 348 A and WO 95/10947 A.

The object of the present invention is therefore to satisfy these and other needs which will become more apparent from the detailed description presented hereinafter.

### SUMMARY

In accordance with the present invention, the disadvantages of the prior art have been found to be overcome by providing pharmaceutical compositions of drugs known as replacement narcotics used in drug addiction therapy, such as methadone and/or its salts, preferably its hydrochloride, in a uniform soft-gel matrix to be taken orally without chewing, whereby the uniform matrix has the shape and size of a pill or capsule, said pharmaceutical compositions comprising in the dry state 30%-75%, preferably 40%-65%, by weight of bovine, porcine, chicken, turkey or fish gelatin, the drug used in the drug addiction therapy in a pharmaceutically effective concentration for this purpose, preferably 0.5%-20% by weight, characterised by containing in the dry state 10%-60% by weight preferably 25%-45% by weight of an adjuvant excipient chosen from the group consisting of polyhydroxy and polyether alcohols, preferably chosen from the group consisting of glycerol, sorbitol/sorbitans, 1,2-propylene glycol, polyethylene glycols, mannitol or mixtures thereof and 1%-10% by weight of water. Among the adjuvant excipients particularly preferred are glycerol and 1,2-propylene glycol.

### DETAILED DESCRIPTION OF THE INVENTION

In particular, the new pharmaceutical composition described herein of drugs used in drug addiction therapy (i.e. so called replacement drugs), in particular methadone and its salts, preferably its hydrochloride, in a uniform soft-gel matrix to be taken orally without chewing, (where the uniform matrix consists of a three dimensional body having the shape and size of a normal pill or capsule intended for oral consumption), said matrix comprising, in the dry state, 30%-75% (preferably 40%-65%) by weight of bovine, porcine, chicken, turkey or fish gelatin, characterised by containing, in the dry state, 10%-60% by weight (preferably 25%-45% by weight) of an adjuvant excipient chosen from the group consisting of polyhydroxy and polyether alcohols, preferably chosen from the group consisting of glycerol, sorbitol/sorbitans, 1,2-propylene glycol, polyethylene glycols, mannitol or mixtures thereof and 1%-10% by weight of water, has been found to possess considerable advantages compared to normal administration of known pharmaceutical forms (capsules with liquid or solid content, or pills) which instead lend themselves to abusive administration. One of the characteristics of the new pharmaceutical formulation described herein is the fact that in said new formulations, the drug used in the drug addiction therapy, in particular methadone hydrochloride, is entirely gelatinised, i.e. uniformly micro-incorporated within the soft-gel matrix.

The new formulations in uniform soft-gel matrices, when compared with the previously known capsules or pills, do not lend themselves to abusive self-administering of the active principle contained therein, other than by slow and very difficult dissolution - assisted in any case by heating and vigorous mechanical agitation - of the soft-gel matrix subjected to the attempted misuse, in comparatively large volumes of water, making it impossible to attain the concentration needed to achieve euphoria on their entry directly into the circulation by intravenous means. Moreover, cooling these solutions, achieved only with the greatest difficulty, tends to restore the gel state which is a characteristic of the starting formulation, and this then renders further not feasible attempts to self-administer intravenously.

These characteristics of the new soft-gel matrix formulations described herein make its abusive administration to unknowing third parties equally difficult. To deter such illicit practices, colorants and/or substances with an unpleasant flavour/odour can be incorporated.

In addition to rendering not feasible intravenous abusive self-administering and the illicit administration to third parties, the new formulations are also more compact and easy to handle than the syrup doses previously used in this field, resulting in a considerable saving along the entire supply chain of legitimate distribution.

Certain characteristics of the pharmaceutical formulation will be examined hereinafter.

The term "dry state" means preferably the state attained by the pharmaceutical formulation after drying at a temperature between 20°C-24°C and 20% relative humidity with continuous change of the surrounding air until a constant weight is achieved, i.e. until two weighings undertaken 24 hours apart do not differ by more than 1 %.

The uniform soft-gel matrices of the present invention contain a drug used in the drug addiction therapy, preferably methadone or its salt, in particular its hydrochloride, in a pharmaceutically acceptable quantity, preferably 0.5%-20% by weight of the dried matrix. In the soft-gel matrices of the present invention, it is particularly preferred that the content of drug used in the drug addiction therapy is 0.5-8.5% by weight.

In the case of methadone hydrochloride, the absolute doses incorporated in a single soft-gel matrix in accordance with the present invention vary between 1 mg and 150 mg, preferably between 5 mg and 100 mg, according to the daily dose prescribed for the scope of the maintenance therapy.

Optionally, the uniform soft-gel matrices of the present invention can possess enteric layers on their exterior formulated in accordance with known techniques in order to substantially degrade in the small intestine.

In addition to (or instead of) possible enteric layers, the uniform soft-gel matrices of the present invention can also have optional further external layers to facilitate ingestion, i.e. being composed of excipients which reduce friction between the matrix and the oesophagus of the patient.

The materials used for obtaining uniform soft-gel matrices of the present invention are the so-called type A or type B gelatins of bovine and porcine origin, or of avian (chicken, turkey) or fish origin normally used in the pharmaceutical art for the production of capsules. In uniform soft-gel matrices of the present invention, the gelatins are present in the dried product from 30% to 75% by weight. Preferably the gelatins are present from 40%-65% by weight. A representative but not exclusive example of a gelatin usable for the scope of the present invention is a gelatin with the following amino acid profile: Glycine: 26%, Alanine: 9%, Isoleucine: 1.5%, Leucine: 3.4%, Valine: 2.5%, Serine: 3.5%, Threonine: 2%, Proline: 16%, Phenylalanine: 2.4%, Tyrosine: 0.8%, Tryptophan: 0%, Methionine: 0.8%, Histidine: 0.8%, Arginine: 9%, Lysine: 5%, Aspartic acid: 6%, Glutamic acid: 11%, Hydroxyproline: 13.5% and Hydroxylysine: 1%. Preferably, the gelatins usable within the scope of the present invention have a particle size distribution between 4 and 100 mesh and a pH between 3 and 10.

The adjuvant excipients usable for obtaining the uniform soft-gel matrices of the present invention are chosen from the group consisting of polyhydroxy and polyether alcohols, preferably chosen from the group consisting of glycerol, sorbitol/sorbitans, 1,2-propylene glycol, polyethylene glycols, mannitol or mixtures thereof. Among these, particularly preferred are glycerol and 1,2-propylene glycol, or mixtures thereof. The adjuvant excipients are contained in the soft-gel matrix in the dry state to the extent of 10%-60% by weight, preferably 25-45% by weight.

The solvent used to obtain the uniform soft-gel matrices of the present invention is water, which remains present in the dried product in a quantity of 1-10% by weight.

A further optionally usable solvent for obtaining uniform soft-gel matrices of the present invention is ethanol which when used remains present in the dried product in a quantity of 0.5-5% by weight.

Further optionally usable components for obtaining the uniform soft-gel matrices of the present invention are excipients, for example all the usual solid pharmaceutically acceptable additives which can be used to modify the release characteristics of the replacement drugs (in particular methadone) from the resulting uniform soft-gel matrix. Further excipients usable for obtaining the uniform soft-gel matrices of the present invention are colorants and/or preservatives, for example parabens, preferably methyl parahydroxybenzoate, ethyl paraoxybenzoate or propyl parahydroxybenzoate.

In accordance with a particularly advantageous aspect of the present invention, the pharmaceutical formulations used in drug dependence therapy in uniform soft-gel matrices can be obtained in accordance with two different procedures which both employ so-called rotary die machines commonly used in pharmaceutical art for the production of classical soft capsules with a liquid or semi-liquid content.

The specific strategies proposed by the procedures of the present invention provide that instead of classic biphasic capsules, i.e. comprising a shell and a contents of different consistencies, "full" capsules are obtained i.e. uniform soft-gel matrices which are perfectly monophasic and therefore unable to be emptied for example with a syringe.

In accordance with the first procedure of the present invention all the components that are necessary to obtain the pharmaceutical composition in the uniform soft-gel matrix of the present invention are mixed together and the mixture is then fed into a forming machine of the rotary die type, which then forms "full" capsules, without injectate. Said "full" capsules make up the pharmaceutical compositions in uniform soft-gel matrices of the present invention.

In accordance with a first variant of the first procedure of the present invention all the components necessary for obtaining the pharmaceutical composition in the uniform soft-gel matrix of the present invention are initially mixed together to obtain a medicated gelatin mix. The medicated gelatin mix is melted and fed into a rotary die encapsulation machine, which then forms the "full" capsules without injectate. Said "full" capsules make up the pharmaceutical compositions in uniform soft-gel matrices of the present invention.

In particular, in accordance with the first variant of the first procedure of the present invention, the following steps are undertaken for the preparation of a pharmaceutical composition of drugs used in drug addiction therapy in a uniform soft-gel matrix:
- preparing a medicated gelatin mix comprising 30-50% by weight of type A or B gelatin of bovine, porcine, turkey, chicken or fish origin, 10-40% by weight of an adjuvant excipient chosen from the group consisting of polyhydroxy and polyether alcohols, 0-10% by weight of ethanol, 20-80% by weight of water and 0.5-15% by weight of the drug used in the drug addiction therapy.
- melting the medicated gelatin mix at a temperature between 30° and 70°C, preferably between 45° and 55°C,
- feeding the medicated gelatin mix into the cavity of the forming rollers of a rotary die encapsulation machine,
- cutting and withdrawing the pharmaceutical composition in uniform soft-gel matrix thus formed, from the rotary die machine, and
- drying the pharmaceutical composition in uniform soft-gel matrix.

Preferably the rotary die machines are operated in an environment at a temperature between 20°C and 24°C at a relative humidity between 5% and 35%, preferably about 20%.

Preferably the pharmaceutical composition in uniform soft-gel matrix obtained as aforesaid is dried at a temperature between 20°C and 24°C at 20% relative humidity with continuous change of the surrounding air until the weight is constant i.e. until two weighings undertaken 24 hours apart do not differ by more than 1%.

If required, further excipients, preservatives and/or colorants can be added to the medicated gelatin mix obtained in the first passage.

In accordance with a second variant of the first procedure of the present invention, some of the components necessary for obtaining the pharmaceutical composition in uniform soft-gel matrix of the present invention, among these the gelatin, are mixed to obtain a gelatin mix. The gelatin mix is melted, a medicated composition containing the active principle is added to thereto to obtain a medicated gelatin mix and the same is fed into a rotary die encapsulation machine, which then forms the "full" capsule, without injectate. Said "full" capsules make up the pharmaceutical compositions in uniform soft-gel matrices of the present invention.

In particular, in accordance with the second variant of the first procedure of the present invention, for the preparation of a pharmaceutical composition of drugs used in the drug addiction therapy in a uniform soft-gel matrix, the following steps are undertaken:
- preparing a gelatin mix comprising 30-50% by weight of type A or B gelatin of bovine, porcine, turkey, chicken or fish origin, 10-40% by weight of an adjuvant excipient chosen from the group consisting of polyhydroxy and polyether alcohols, 0-10% by weight of ethanol and 20-60% by weight of water,
- melting the gelatin mix at a temperature between 30° and 80°C, preferably between 40° and 65°C,
- when melting is complete, lowering the gelatin mix temperature to 45° ± 5°C and adding a medicated mixture comprising the drug used in the drug addiction therapy in the required quantity and a liquid adjuvant excipient in accordance with the present invention, for example glycerol, 1,2-propylene glycol or an aqueous solution of sorbitol/sorbitans, the quantity of medicated mixture corresponding to 5%-10% by weight of the gelatin mix, to obtain a medicated gelatin mix,
- feeding the medicated gelatin mix into the cavity of the forming rollers of a rotary die encapsulation machine,
- cutting and withdrawing the pharmaceutical composition in uniform soft-gel matrix thus formed, from the rotary die machine, and
- drying the pharmaceutical composition in uniform soft-gel matrix.

If required, further excipients, preservatives and/or colorants can be added to the gelatin mix obtained in the first passage and/or to the medicated mixture added in the third passage.

Preferably, the rotary die type machines are operated in an environment with a temperature between 20°C and 24°C at a relative humidity between 5% and 35%, preferably around 20%. Preferably, the pharmaceutical composition in uniform soft-gel matrix obtained as aforesaid is dried at a temperature between 20°C and 24°C at 20% relative humidity with continuous change of the surrounding air until a constant weight is achieved, i.e. until two weighings undertaken 24 hours apart do not differ by more than 1%.

A second procedure (which is particularly preferred) for obtaining pharmaceutical formulations of drugs used in drug addiction therapy in uniform soft-gel matrices of the present invention, comprises dissolving/suspending the active principle and any excipients in a liquid carrier, thus giving the so called "medicated injectate" which is then injected into the interior of the gelatin mix at the moment of matrix formation. The components of the gelatin mix and, respectively, of the medicated injectate, are specifically balanced to enable uniform diffusion of the medicated injectate within the interior of the matrix, without altering its monophasic structure. As a consequence, again with the second procedure of the present invention, one does not obtain the usual soft capsules filled with a liquid, semi-liquid or paste-like phase, but one obtains instead a uniform soft-gel matrix comprising the active principle.

In particular, for the preparation of a pharmaceutical composition of drugs used for drug addiction therapy in a uniform soft-gel matrix in accordance with the second procedure of the present invention, the following steps are undertaken:
- preparing a gelatin mix comprising 30-50% by weight of type A or B gelatin of bovine, porcine, turkey, chicken or fish origin, 10-40% by weight of an adjuvant excipient chosen from the group consisting of polyhydroxy and polyether alcohols, 0-10% by weight of ethanol and 20-80% by weight of water (preferably 25%-40% of water),
- melting the gelatin mix at a temperature between 30° and 80°C, preferably between 40° and 65°C,
- feeding the gelatin mix into the cavity of the forming rollers of a rotary die encapsulation machine; injecting through the foreseen injector, on closure of the cavity, a quantity of medicated injectate corresponding to from 1% to 60% by weight, preferably from 10% to 50% by weight, of the quantity of gelatin mix positioned in the cavity, said medicated injectate comprising
   20-60% by weight of an adjuvant excipient chosen from the group consisting of polyhydroxy and polyether alcohols,
   0-50% by weight of ethanol,
   10-50% by weight of water
   0-55% by weight of gelatin
   the required quantity, in weight, of the drug used in the drug addiction therapy,
- cutting and withdrawing the pharmaceutical composition in the uniform soft-gel matrix thus formed, from the rotary die machine, and
- drying the pharmaceutical composition in the uniform soft-gel matrix.

Preferably, the medicated injectate comprises 25%-42% by weight of an adjuvant excipient chosen from the group consisting of polyhydroxy and polyether alcohols, 0%-30% by weight of ethanol, 10%-45% by weight of water, 10%-45% by weight of gelatin, and and a sufficient quantity in weight of the drug used in the drug addiction therapy, preferably methadone or its salts, in particular methadone hydrochloride.

Even more preferably, the medicated injectate comprises 30%-36% by weight of an adjuvant excipient chosen from the group consisting of polyhydroxy and polyether alcohols, 10%-26% by weight of water, 25-42% of gelatin, and a sufficient quantity in weight of the drug used in the drug addiction therapy, preferably methadone or its salts, in particular methadone hydrochloride.

If required, further excipients, preservatives and/or colorants can be added to the gelatin mix obtained in the first step and/or to the medicated injectate added in the third step.

Preferably, the rotary die machines are operated in an environment having a temperature between 20°C and 24°C at relative humidity between 5% and 35%, preferably around 20%. Preferably, the pharmaceutical composition in uniform soft-gel matrix obtained as aforesaid is dried at a temperature between 20°C and 24°C at 20% relative humidity with continuous change of the surrounding air until a constant weight is achieved, i.e. until two weighings undertaken 24 hours apart do not differ by more than 1%.

As previously mentioned, under the aforegiven production conditions, the medicated injectate never remains a liquid or paste-like phase, distinguishable from the gelatin phase, but diffuses uniformly within the gelatin mix, to give a uniform soft-gel matrix which can be taken orally.

It is hence apparent that the pharmaceutical compositions of drugs used in drug addiction therapy in uniform soft-gel matrix in accordance with both aspects of the present invention contain the drug used in the drug addiction therapy in a uniformly micro-incorporated gelatinized form, and can be easily divided by the patient - in contrast to normal soft capsules of liquid or semi-liquid content - so that the individual dosage prescribed by the doctor can be adapted if necessary.

### EXPERIMENTAL PART

Some examples of formulations in accordance with the present invention are given hereinafter:

### Examples 1-6

The following formulations of the present invention were obtained in accordance with the second procedure of the present invention:
In the tables of examples 1-6, the first three columns refer to the initial situation, i.e. prior to injection:
- First column: composition in respective percentages of the medicated injectate and the gelatin mix.
- Second column: total quantity of medicated injectate and gelatin mix in mg.
- Third column: quantity of each ingredient in mg/ uniform soft-gel matrix for the just formed matrix.
The last three columns refer to the situation after injection:
- Fourth column: quantity in mg/matrix of each ingredient in the dried soft-gel matrix.
- Fifth and sixth column: percentages of each ingredient in the dried soft-gel matrix.
In the dried matrices (at a temperature between 20°C and 24°C at 20% relative humidity with continuous change of the surrounding air until a constant weight is achieved, i.e. until two weighings undertaken 24 hours apart do not differ by more than 1%), the weight decreases due to the almost complete removal of the remaining water bound to the gelatin in the quantities specified.

| **Example1** | | | | | | |
|---|---|---|---|---|---|---|
| | Initial | | | Final | | |
| **Medicated injectate** | % | mg/matrix | mg/matrix | Mg/soft-gel matrix | % after drying | % after drying |
| Hydrolyzed gelatin | 35 | 250 | 87.5 | 87.50 | 13.71 | |
| Gelatin | 5 | 250 | 12.5 | 12.50 | 1.96 | |
| Glycerol | 35 | 250 | 87.5 | 87.50 | 13.71 | |
| Propylene glycol | | 250 | 0 | 0.00 | 0.00 | |
| Water | 23 | 250 | 55.75 | 0.00 | 0.00 | |
| Methadone hydrochloride | 2 | 250 | 5 | 5.00 | 0.78 | 0.78 |
| Total | 100 | 250 | 250 | | | |
| **Gelatin mix** | | | | | 0.00 | |
| Gelatin | 47 | 625 | 293.75 | 293.75 | 46.03 | 61.70 |
| Glycerol | 18 | 625 | 112.5 | 112.50 | 17.63 | 31.34 |
| Water | 35 | 625 | 218.75 | 39.38 | 6.17 | 6.17 |
| Total | 100 | 625 | 625 | 638.13 | 100.00 | 100.00 |

| **Example 2** | | | | | | |
|---|---|---|---|---|---|---|
| | | Initial | | | Final | |
| **Medicated injectate** | % | mg/matrix | mg/matrix | Mg/soft-gel matrix | % after drying | % after drying |
| Hydrolyzed gelatin | 35 | 500 | 175 | 175 | 15.19 | |
| Gelatin | 5 | 500 | 25 | 25 | 2.17 | |
| Glycerol | 0 | 500 | 0 | 0 | 0.00 | |
| Propylene glycol | 35 | 500 | 175 | 175 | 15.19 | 15.19 |
| Water | 13 | 500 | 65 | 0 | 0.00 | |
| Methadone hydrochloride | 12 | 500 | 60 | 60 | 5.21 | 5.21 |
| Total | 100 | | 500 | | | |
| **Gelatin mix** | | | 0 | 0 | 0.00 | |
| Gelatin 200 bloom | 47 | 1000 | 470 | 470 | 40.80 | 58.16 |
| Glycerol | 18 | 1000 | 180 | 180 | 15.63 | 15.63 |
| Water | 35 | 1000 | 350 | 67 | 5.82 | 5.82 |
| Total | 100 | | 1000 | 1152 | 100 | 100 |

| **Example 3** | | | | | | |
|---|---|---|---|---|---|---|
| | | Initial | | | Final | |
| **Medicated injectate** | % | mg/matrix | mg/matrix | Mg/soft-gelmatrix | % after drying | % after drying |
| Hydrolyzed gelatin | 35.3 | 425 | 150 | 150 | 14.05 | |
| Gelatin 80 bloom | 5.88 | 425 | 25 | 25 | 2.34 | |
| Glycerol 85% | 20 | 425 | 85 | 85 | 7.96 | |
| Propylene glycol | 14.1 | 425 | 60 | 60 | 5.62 | 5.62 |
| Water | 15.3 | 425 | 65 | 0 | 0.00 | |
| Methadone hydrochloride | 9.41 | 425 | 40 | 40 | 3.75 | 3.75 |
| Total | 100 | | 425 | | | |

| **Gelatin mix** | | | | | | |
|---|---|---|---|---|---|---|
| Gelatin 200 bloom | 40 | 1000 | 400 | 400 | 37.47 | 53.86 |
| Glycerol | 25 | 1000 | 250 | 250 | 23.42 | 31.38 |
| Water | 35 | 1000 | 350 | 57.5 | 5.39 | 5.39 |
| Total | 100 | | 1000 | 1067.5 | 100.00 | 100.00 |

| **Example 4** | | | | | | |
|---|---|---|---|---|---|---|
| | | Initial | | | Final | |
| **Medicated injectate** | % | mg/matrix | mg/matrix | Mg/soft-gel matrix | % after drying | % after drying |
| Hydrolyzed gelatin | 26.1 | 575 | 150 | 150 | 12.32 | |
| Gelatin 80 bloom | 4.35 | 575 | 25 | 25 | 2.05 | |
| Glycerol 85% | 14.8 | 575 | 85 | 85 | 6.98 | |
| Propylene glycol | 26.1 | 575 | 150 | 150 | 12.32 | 12.32 |
| Water | 11.3 | 575 | 65 | 0 | 0.00 | |
| Methadone hydrochloride | 17.4 | 575 | 100 | 100 | 8.21 | 8.21 |
| Total | 100 | | 575 | | | |

| **Gelatin mix** | | | | | | |
|---|---|---|---|---|---|---|
| Gelatin 200 bloom | 40 | 1000 | 400 | 400 | 32.85 | 47.23 |
| Glycerol | 25 | 1000 | 250 | 250 | 20.53 | 27.52 |
| Water | 35 | 1000 | 350 | 57.5 | 4.72 | 4.72 |
| Total | 100 | | 1000 | 1217.5 | 100.00 | 100 |

| **Example 5** | | | | | | |
|---|---|---|---|---|---|---|
| | | Initial | | | Final | |
| **Medicated injectate** | % | mg/matrix | mg/matrix | Mg/soft-gel matrix | % after drying | % after drying |
| Hydrolyzed gelatin | 35 | 250 | 87.5 | 87.50 | 13.71 | |
| Gelatin | 5 | 250 | 12.5 | 12.50 | 1.96 | |
| Glycerol | 35 | 250 | 87.5 | 87.50 | 13.71 | |
| Propylene glycol | | 250 | 0 | 0.00 | 0.00 | |
| Water | 25 | 250 | 62.5 | 0.00 | 0.00 | |
| Methadone hydrochloride | 2 | 250 | 5 | 5.00 | 0.78 | 0.78 |
| Total | 102 | 250 | 250 | | | |
| **Gelatin mix** | | | | | 0.00 | |
| Gelatin | 47 | 625 | 293.75 | 293.75 | 46.03 | 61.70 |
| sorbitol/sorbitans 85 % | 18 | 625 | 112.5 | 112.50 | 17.63 | 31.34 |
| Water | 35 | 625 | 218.75 | 39.38 | 6.17 | 6.17 |
| Total | 100 | 625 | 625 | 638.13 | 100.00 | 100.00 |

| **Example 6** | | | | | | |
|---|---|---|---|---|---|---|
| | | Initial | | | Final | |
| **Medicated injectate** | % | mg/matrix | mg/matrix | Mg/soft-gel matrix | % after drying | % after drying |
| Hydrolyzed gelatin | 35 | 500 | 175 | 175 | 15.19 | |
| Gelatin | 5 | 500 | 25 | 25 | 2.17 | |
| Glycerol | 0 | 500 | 0 | 0 | 0.00 | |
| Propylene glycol | 35 | 500 | 175 | 175 | 15.19 | 15.19 |
| Water | 13 | 500 | 65 | 0 | 0.00 | |
| Methadone hydrochloride | 12 | 500 | 60 | 60 | 5.21 | 5.21 |
| Total | 100 | | 500 | | | |
| **Gelatin mix** | | | 0 | 0 | 0.00 | |
| Gelatin 200 bloom | 47 | 1000 | 470 | 470 | 40.80 | 58.16 |
| sorbitol/sorbitans 85 % | 18 | 1000 | 180 | 180 | 15.63 | 15.63 |
| Water | 35 | 1000 | 350 | 67 | 5.82 | 5.82 |
| Total | 100 | | 1000 | 1152 | 100 | 100 |

### Example 7

Obtaining formulations of drugs used in drug addiction therapy in uniform soft-gel matrices of the present invention, specifically in accordance with the first variant of the first procedure:
Introducing the medicated gelatin mix as aforedefined into a stainless steel reactor equipped with heating system, mixer and apparatus for operating under vacuum and under pressure; melting the mass thus obtained at around 50°C under constant stirring and under vacuum. When fully melted the mix is discharged into suitable temperature-controlled stainless steel vessels, where it is maintained at about 45°C. From here, the mix is fed to a rotary die encapsulating machine, for example a MKSJ Encapsulating Machine (SEN JIN SDN.BHD).
In detail, the hot gelatin mix feeds into two dispensers on the machine, which form two gelatin films, of determined and constant thickness, on two air cooled drums. The two films pass through two concentrically rotating encapsulating rollers, on which a special heated wedge rests, known as an injector segment, but not used in this procedure. On passing through the rollers, the encapsulating cavities form uniform soft-gel matrices from the two gelatin films. The uniform soft-gel matrices which are cut off fall under the forming rollers into rotating baskets from which, after remaining for some hours, they are tipped into trays for drying.
Where the starting point is a non-medicated gelatin mix, i.e. in the second variant of the first procedure, the process is similar but the melting temperature is higher, around 65°C. When fully melted the mix is discharged into suitable stainless steel temperature controlled vessels, where it is maintained at about 45°C for the required time. The process continues by adding the medicated mixture, then homogenising, from where the mix is fed, preferably within one hour, to a rotary die encapsulating machine which completes the formation of uniform soft-gel matrices as aforedescribed with regard to the first variant of the first procedure.
Obtaining drug formulations which are usable for drug addiction therapy in uniform soft-gel matrices in accordance with the present invention, in particular according to the second procedure:
   The process corresponds to the second variant of the first procedure, without addition of the medicated mixture to the mix before being fed into the machine. That is to say, the hot gelatin mix feeds two dispensers on the machine, which form two gelatin films, of determined and constant thickness, on two air cooled drums. The two films pass through two concentrically rotating encapsulating rollers, on which a special heated wedge rests, known as an injector segment. The medicated injectate is fed directly to a dispensing pump which has precision syringes sliding in reciprocating manner, to feed the injector segment through small tubes, which injects a quantity of medicated injectate into the gelatin mix contained in the cavities of the two forming rollers. The medicated injectate diffuses through the gelatin mix, hence forming the uniform soft-gel matrices which are cut off and fall under the forming rollers into rotating baskets from which, after remaining for some hours, they are tipped into trays for drying.

## Claims

1. A pharmaceutical composition of drugs known as replacement narcotics used in drug addiction therapy, preferably of methadone or its salts, in an effective pharmaceutical concentration, preferably 0.5%-20% by weight, in a uniform soft-gel matrix that can be taken orally without chewing, in which the matrix has the shape and size of a pill or capsule, the pharmaceutical composition comprising, in the dry state and in addition to the drug used in the drug addiction therapy, 30%-75% by weight of bovine, porcine, chicken, turkey or fish gelatin **characterised by** containing in the dry state 10%-60% by weight of an adjuvant excipient chosen from the group consisting of polyhydroxy and polyether alcohols and 1 %-10% by weight of water.

2. Pharmaceutical composition as claimed in claim 1, **characterised by** containing in the dry state 25%-45% by weight of an adjuvant excipient chosen from the group consisting of polyhydroxy and polyether alcohols or mixtures thereof.

3. Pharmaceutical composition as claimed in claim 1 or 2, **characterised by** containing, in the dry state, 40%-65% of gelatin.

4. Pharmaceutical composition in accordance with one or more of the preceding claims, **characterised by** containing an adjuvant excipient chosen from the group consisting of glycerol, sorbitol/sorbitans, 1,2-propylene glycol, polyethylene glycols, mannitol or mixtures thereof.

5. Pharmaceutical composition as claimed in claim 4, **characterised by** containing an adjuvant excipient chosen from the group consisting of glycerol and 1,2-propylene glycol or mixtures thereof.

6. Pharmaceutical composition as claimed in one or more of the preceding claims, wherein the gelatin has a pH between 3 and 10.

7. Pharmaceutical composition as claimed in one or more of the preceding claims, **characterised by** containing 0.5-5% by weight of ethanol.

8. Pharmaceutical composition in accordance with one or more of the preceding claims, **characterised by** comprising further excipients, for example solid additives to modify the release characteristics of the drugs used in the drug addiction therapy from the uniform soft-gel matrix, or preservatives and/or colorants.

9. Pharmaceutical composition in accordance with one or more of the preceding claims **characterised in that** the drug used in the drug addiction therapy is methadone hydrochloride.

10. Pharmaceutical composition as claimed in one or more of the preceding claims provided externally with enteric layers and/or layers to facilitate ingestion.

11. Method for obtaining the pharmaceutical compositions as claimed in claim 1, comprising the following steps:
- preparing a medicated gelatin mix comprising 30-50% by weight of type A or B gelatin of bovine, porcine, turkey, chicken or fish origin, 10-40% by weight of an adjuvant excipient chosen from the group consisting of polyhydroxy and polyether alcohols, 0-10% by weight of ethanol, 20-80% by weight of water and 0.5-15% by weight of the drug used in the drug addiction therapy,
- melting the medicated gelatin mix at a temperature between 30° and 70°C, preferably between 45° and 65°C,
- feeding the medicated gelatin mix into the cavity of the forming rollers of a rotary die encapsulation machine,
- cutting and withdrawing the pharmaceutical composition in uniform soft-gel matrix thus formed, from the rotary die machine, and
- drying the pharmaceutical composition in uniform soft-gel matrix.

12. Method for obtaining the pharmaceutical compositions as claimed in claim 1, comprising the following steps:
- preparing a gelatin mix comprising 30-50% by weight of type A or B gelatin of bovine, porcine, turkey, chicken or fish origin, 10-40% by weight of an adjuvant excipient chosen from the group consisting of polyhydroxy and polyether alcohols, 0-10% by weight of ethanol and 20-60% by weight of water,
- melting the gelatin mix at a temperature between 30° and 80°C, preferably between 40° and 65°C,
- when melting is complete, lowering the gelatin mix temperature to 45° ± 5°C and adding a medicated mixture comprising the drug used in the drug addiction therapy in the required quantity and an adjuvant excipient chosen from the group consisting of glycerol, 1,2-propylene glycol and an aqueous solution of sorbitol/sorbitans, or a mixture thereof, the quantity of medicated mixture corresponding to 5%-10% by weight of the gelatin mix, to obtain a medicated gelatin mix,
- feeding the medicated gelatin mix into the cavity of the forming rollers of a rotary die encapsulation machine,
- cutting and withdrawing the pharmaceutical composition in uniform soft-gel matrix thus formed, from the rotary die machine, and
- drying the pharmaceutical composition in uniform soft-gel matrix.

13. Method for obtaining the pharmaceutical compositions as claimed in claim 1, comprising the following passages:
- preparing a gelatin mix comprising 30-50% by weight of type A or B gelatin of bovine, porcine, turkey, chicken or fish origin, 10-40% by weight of an adjuvant excipient chosen from the group consisting of polyhydroxy and polyether alcohols, 0-10% by weight of ethanol and 20-80% by weight of water,
- melting the gelatin mix at a temperature between 30° and 80°C, preferably between 40° and 65°C,
- feeding the gelatin mix into the cavity of the forming rollers of a rotary die encapsulation machine; injecting by a foreseen injector, on closure of the cavity, a quantity of medicated injectate corresponding to 1-60% by weight, preferably from 10% to 50% by weight, of the quantity of gelatin mix positioned in the cavity, said medicated injectate comprising
20-60% by weight of an adjuvant excipient chosen from the group consisting of polyhydroxy and polyether alcohols,
0-50% by weight of ethanol,
10-50% by weight of water,
0-55% by weight of gelatin,
the required quantity, in weight, of the drug used in the drug addiction therapy,
- cutting and withdrawing the pharmaceutical composition in uniform soft-gel matrix thus formed, from the rotary die machine, and
- drying the pharmaceutical composition in uniform soft-gel matrix.

14. Method as claimed in claim 13, wherein the medicated injectate comprises 25%-42% by weight of an adjuvant excipient chosen from the group consisting of polyhydroxy and polyether alcohols, 0%-30% by weight of ethanol, 10%-45% by weight of water, 10%-45% by weight of gelatin and the required quantity, in weight, of the drug used in the drug addiction therapy.

## Patentansprüche

1. Pharmazeutische Zusammensetzung von Arzneimitteln, welche als in der Therapie von Drogensucht verwendete Ersatzdrogen bekannt sind, vorzugsweise von Methadon oder dessen Salzen, in einer wirksamen pharmazeutischen Konzentration von vorzugsweise 0,5 % bis 20 Gew.-% in einer gleichmäßigen Weichgelmatrix, welche ohne Kauen oral eingenommen werden kann, wobei die Matrix die Form und die Größe einer Pille oder Kapsel aufweist, wobei die pharmazeutische Zusammensetzung in dem Trockenzustand und zusätzlich zu dem in der Therapie von Drogensucht eingesetzten Arzneimittel 30 % bis 75 Gew.-% Rinder-, Schwein-, Huhn-, Truthahn- oder Fischgelatine enthält, **dadurch gekennzeichnet, dass** diese in dem Trockenzustand 10 % bis 60 Gew.-% eines adjuvanten Hilfsmittels ausgewählt aus der Gruppe bestehend aus Polyhydroxy- und Polyetheralkoholen sowie 1 % bis 10 Gew.-% Wasser enthält.

2. Pharmazeutische Zusammensetzung nach Anspruch 1, **dadurch gekennzeichnet, dass** diese in dem Trockenzustand 25 % bis 45 Gew.-% eines adjuvanten Hilfsmittels ausgewählt aus der Gruppe bestehend aus Polyhydroxy- und Polyetheralkoholen oder Mischungen hiervon enthält.

3. Pharmazeutische Zusammensetzung nach Anspruch 1 oder 2, **dadurch gekennzeichnet, dass** diese in dem Trockenzustand 40 % bis 65 % Gelatine enthält.

4. Pharmazeutische Zusammensetzung nach einem oder mehreren der vorhergehenden Ansprüche, **dadurch gekennzeichnet, dass** diese ein adjuvantes Hilfsmittel ausgewählt aus der Gruppe bestehend aus Glycerin, Sorbit/Sorbitanen, 1,2-Propylenglykol, Polyethylenglykolen, Mannit oder Mischungen hiervon enthält.

5. Pharmazeutische Zusammensetzung nach Anspruch 4, **dadurch gekennzeichnet, dass** diese ein adjuvantes Hilfsmittel ausgewählt aus der Gruppe bestehend aus Glycerin und 1,2-Propylenglykol oder Mischungen hiervon enthält.

6. Pharmazeutische Zusammensetzung nach einem oder mehreren der vorhergehenden Ansprüche, wobei die Gelatine einen pH-Wert zwischen 3 und 10 aufweist.

7. Pharmazeutische Zusammensetzung nach einem oder mehreren der vorhergehenden Ansprüche, **dadurch gekennzeichnet, dass** diese 0,5 bis 5 Gew.-% Ethanol enthält.

8. Pharmazeutische Zusammensetzung nach einem oder mehreren der vorhergehenden Ansprüche, **dadurch gekennzeichnet, dass** diese ferner Hilfsmittel, beispielsweise Feststoffadditive, um die Freisetzungseigenschaften der Arzneimittel, welche in der Therapie von Drogensucht eingesetzt werden, aus der gleichmäßigen Weichgelmatrix zu verändern, oder Konservierungsstoffe und/oder Farbstoffe enthält.

9. Pharmazeutische Zusammensetzung nach einem oder mehreren der vorhergehenden Ansprüche, **dadurch gekennzeichnet, dass** das in der Therapie von Drogensucht eingesetzte Arzneimittel Methadonhydrochlorid ist.

10. Pharmazeutische Zusammensetzung nach einem oder mehreren der vorhergehenden Ansprüche, wobei diese außen mit Magensaft resistenten Schichten und/oder Schichten zum Erleichtern der Aufnahme ausgestattet sind.

11. Verfahren zum Herstellen von pharmazeutischen Zusammensetzungen nach Anspruch 1, umfassend die nachfolgenden Schritte:
- Herstellen einer Arzneimittel enthaltenden Gelatinemischung enthaltend 30 bis 50 Gew.-% von aus Rind, Schwein, Truthahn, Huhn oder Fisch stammender Gelatine vom Typ A oder B, 10 bis 40 Gew.-% eines adjuvanten Hilfsmittels ausgewählt aus der Gruppe bestehend aus Polyhydroxy- und Polyetheralkoholen, 0 bis 10 Gew.-% Ethanol, 20 bis 80 Gew.-% Wasser und 0,5 bis 15 Gew.-% des in der Therapie von Drogensucht eingesetzten Arzneimittels,
- Schmelzen der Arzneimittel enthaltenden Gelatinemischung bei einer Temperatur zwischen 30 °C und 70 °C, vorzugsweise zwischen 45 °C und 65 °C,
- Zuführen der Arzneimittel enthaltenden Gelatinemischung in den Hohlraum von Formwalzen einer Rotary-Die-Verkapselungsmaschine,
- Schneiden und Entnehmen der pharmazeutischen Zusammensetzung in der so gebildeten gleichmäßigen Weichgelmatrix aus der Rotary-Die-Maschine und
- Trocknen der pharmazeutischen Zusammensetzung in gleichmäßiger Weichgelmatrix.

12. Verfahren zum Herstellen von pharmazeutischen Zusammensetzungen nach Anspruch 1, umfassend die nachfolgenden Schritte:
- Herstellen einer Gelatinemischung, welche 30 bis 50 Gew.-% von aus Rind, Schwein, Truthahn, Huhn oder Fisch stammender Gelatine vom Typ A oder B, 10 bis 40 Gew.-% eines adjuvanten Hilfsmittels ausgewählt aus der Gruppe bestehend aus Polyhydroxy- und Polyetheralkoholen, 0 bis 10 Gew.-% Ethanol und 20 bis 60 Gew.-% Wasser enthält,
- Schmelzen der Gelatinemischung bei einer Temperatur zwischen 30°C und 80 °C, vorzugsweise zwischen 40 °C und 65 °C,
- Verringern der Temperatur der Gelatinemischung auf 45 °C ± 5 °C, wenn das Schmelzen beendet ist, und Zugabe einer Arzneimittel enthaltenden Mischung enthaltend das in der Therapie von Drogensucht eingesetzte Arzneimittel in der benötigten Menge und eines adjuvanten Hilfsmittels ausgewählt aus der Gruppe bestehend aus Glycerin, 1,2-Propylenglykol und einer wässrigen Lösung von Sorbit/Sorbitanen oder einer Mischung hiervon, wobei die Menge der Arzneimittel enthaltenden Mischung 5 % bis 10 Gew.-% der Gelatinemischung entspricht, um eine Arzneimittel enthaltende Gelatinemischung zu erhalten,
- Zuführen der Arzneimittel enthaltenden Gelatinemischung in den Hohlraum von Formwalzen einer Rotary-Die-Verkapselungsmaschine,
- Schneiden und Entnehmen der pharmazeutischen Zusammensetzung in der so gebildeten gleichmäßigen Weichgelmatrix aus der Rotary-Die-Maschine und
- Trocknen der pharmazeutischen Zusammensetzung in gleichmäßiger Weichgelmatrix.

13. Verfahren zum Herstellen von pharmazeutischen Zusammensetzungen nach Anspruch 1, umfassend die nachfolgenden Schritte:
- Herstellen einer Gelatinemischung, welche 30 bis 50 Gew.-% von aus Rind, Schwein, Truthahn, Huhn oder Fisch stammender Gelatine vom Typ A oder B, 10 bis 40 Gew.-% eines adjuvanten Hilfsmittels ausgewählt aus der Gruppe bestehend aus Polyhydroxy- und Polyetheralkoholen, 0 bis 10 Gew.-% Ethanol und 20 bis 80 Gew.-% Wasser enthält,
- Schmelzen der Gelatinemischung bei einer Temperatur zwischen 30 °C und 80 °C, vorzugsweise zwischen 40 °C und 65 °C,
- Zuführen der Gelatinemischung in den Hohlraum von Formwalzen einer Rotary-Die-Verkapselungsmaschine; Injizieren einer 1 bis 60 Gew.-%, vorzugsweise 10 bis 50 Gew.-%, der Menge der in dem Hohlraum positionierten Gelatinemischung entsprechenden Menge von Arzneimittel enthaltendem Injektat durch einen auf dem Verschluss des Hohlraums vorgesehenen Injektor, wobei das Arzneimittel enthaltende Injektat umfasst:
20 bis 60 Gew.-% eines adjuvanten Hilfsmittels ausgewählt aus der Gruppe bestehend aus Polyhydroxy- und Polyetheralkoholen,
0 bis 50 Gew.-% Ethanol,
10 bis 50 Gew.-% Wasser,
0 bis 55 Gew.-% Gelatine,
die benötige Menge, in Gew.-%, des in der Therapie von Drogensucht eingesetzten Arzneimittels,
- Schneiden und Entnehmen der pharmazeutischen Zusammensetzung in der so gebildeten gleichmäßigen Weichgelmatrix aus der Rotary-Die-Maschine und
- Trocknen der pharmazeutischen Zusammensetzung in gleichmäßiger Weichgelmatrix.

14. Verfahren nach Anspruch 13, wobei das Arzneimittel enthaltende Injektat 25 % bis 42 Gew.-% eines adjuvanten Hilfsmittels ausgewählt aus der Gruppe bestehend aus Polyhydroxy- und Polyetheralkoholen, 0 % bis 30 Gew-% Ethanol, 10 % bis 45 Gew-% Wasser, 10 % bis 45 Gew.-% Gelatine und die benötigte Menge, in Gew.-%, des in der Therapie von Drogensucht eingesetzten Arzneimittels enthält.

## Revendications

1. Composition pharmaceutique de médicaments connus comme des narcotiques de remplacement utilisés dans une thérapie de narcomanie, de préférence de méthadone ou de ses sels, dans une concentration pharmaceutique efficace, de préférence 0,5 % à 20 % en poids, dans une matrice de capsule molle uniforme qui peut être prise par voie orale sans mâcher, où la matrice a la forme et la taille d'une pilule ou d'une capsule, la composition pharmaceutique comprenant, à l'état sec et en plus du médicament utilisé dans la thérapie de narcomanie, 30 % à 75 % en poids de gélatine bovine, porcine, de poulet, de dinde ou de poisson, **caractérisée par le fait qu'**elle contient à l'état sec 10 % à 60 % en poids d'un excipient adjuvant choisi dans le groupe constitué d'alcools polyhydroxylés et de polyéther-alcools et 1 % à 10 % en poids d'eau.

2. Composition pharmaceutique selon la revendication 1, **caractérisée par le fait qu'**elle contient à l'état sec 25 % à 45 % en poids d'un excipient adjuvant choisi dans le groupe constitué d'alcools polyhydroxylés et de polyéther-alcools ou de mélanges de ceux-ci.

3. Composition pharmaceutique selon la revendication 1 ou 2, **caractérisée par le fait qu'**elle contient, à l'état sec, 40 % à 65 % de gélatine.

4. Composition pharmaceutique selon une ou plusieurs des revendications précédentes, **caractérisée par le fait qu'**elle contient un excipient adjuvant choisi dans le groupe constitué de glycérol, sorbitol/sorbitanes, 1,2-propylène glycol, polyéthylène glycols, mannitol ou de mélanges de ceux-ci.

5. Composition pharmaceutique selon la revendication 4, **caractérisée par le fait qu'**elle contient un excipient adjuvant choisi dans le groupe constitué de glycérol et de 1,2-propylène glycols ou de mélanges de ceux-ci.

6. Composition pharmaceutique selon une ou plusieurs des revendications précédentes, dans laquelle la gélatine a un pH entre 3 et 10.

7. Composition pharmaceutique selon une ou plusieurs des revendications précédentes, **caractérisée par le fait qu'**elle contient 0,5 à 5 % en poids d'éthanol.

8. Composition pharmaceutique selon une ou plusieurs des revendications précédentes, **caractérisée par le fait qu'**elle comprend d'autres excipients, par exemple, des additifs solides pour modifier les caractéristiques de libération des médicaments utilisés dans la thérapie de narcomanie à partir de la matrice de capsule molle uniforme, ou des conservateurs et/ou des colorants.

9. Composition pharmaceutique selon une ou plusieurs des revendications précédentes, **caractérisée en ce que** le médicament utilisé dans la thérapie de narcomanie est le chlorhydrate de méthadone.

10. Composition pharmaceutique selon une ou plusieurs des revendications précédentes, fournie du côté externe avec des couches gastro-résistantes et/ou des couches pour faciliter l'ingestion.

11. Procédé d'obtention des compositions pharmaceutiques selon la revendication 1, comprenant les étapes suivantes :
- la préparation d'un mélange de gélatine médicamenteuse comprenant 30 à 50 % en poids de gélatine de type A ou B d'origine bovine, porcine, de dinde, de poulet ou de poisson, 10 à 40 % en poids d'un excipient adjuvant choisi dans le groupe constitué d'alcools polyhydroxylés et de polyéther-alcools, 0 à 10 % en poids d'éthanol, 20 à 80 % en poids d'eau et 0,5 à 15 % en poids du médicament utilisé dans la thérapie de narcomanie,
- la fusion du mélange de gélatine médicamenteuse à une température entre 30 et 70°C, de préférence entre 45 et 65°C,
- le versement du mélange de gélatine médicamenteuse dans la cavité des rouleaux de confection d'une machine d'encapsulation à matrice rotative,
- le découpage et le retrait de la composition pharmaceutique dans une matrice de capsule molle uniforme ainsi formée, à partir de la machine à matrice rotative, et
- le séchage de la composition pharmaceutique dans la matrice de capsule molle uniforme.

12. Procédé d'obtention des compositions pharmaceutiques selon la revendication 1, comprenant les étapes suivantes :
- la préparation d'un mélange de gélatine comprenant 30 à 50 % en poids de gélatine de type A ou B d'origine bovine, porcine, de dinde, de poulet ou de poisson, 10 à 40 % en poids d'un excipient adjuvant choisi dans le groupe constitué d'alcools polyhydroxylés et de polyéther-alcools, 0 à 10 % en poids d'éthanol et 20 à 60 % en poids d'eau,
- la fusion du mélange de gélatine à une température entre 30 et 80°C, de préférence entre 40 et 65°C,
- lorsque la fusion est complète, l'abaissement de la température du mélange de gélatine à 45 ± 5°C et l'addition d'un mélange médicamenteux comprenant le médicament utilisé dans la thérapie de la narcomanie dans la quantité requise et un excipient adjuvant choisi dans le groupe constitué de glycérol, de 1,2 propylène glycol et d'une solution aqueuse de sorbitol/sorbitanes, ou d'un mélange de ceux-ci, la quantité du mélange médicamenteux correspondant à 5 % à 10 % en poids du mélange de gélatine, pour obtenir un mélange de gélatine médicamenteuse,
- le versement du mélange de gélatine médicamenteuse dans la cavité des rouleaux de confection d'une machine d'encapsulation à matrice rotative,
- le découpage et le retrait de la composition pharmaceutique dans une matrice de capsule molle uniforme ainsi formée, à partir de la machine à matrice rotative, et
- le séchage de la composition pharmaceutique dans la matrice de capsule molle uniforme.

13. Procédé d'obtention des compositions pharmaceutiques selon la revendication 1, comprenant les étapes suivantes :
- la préparation d'un mélange de gélatine comprenant 30 à 50 % en poids de gélatine de type A ou B d'origine bovine, porcine, de dinde, de poulet ou de poisson, 10 à 40 % en poids d'un excipient adjuvant choisi dans le groupe constitué d'alcools polyhydroxylés et de polyéther-alcools, 0 à 10 % en poids d'éthanol et 20 à 80 % en poids d'eau,
- la fusion du mélange de gélatine à une température entre 30 et 80°C, de préférence entre 40 et 65°C,
- le versement du mélange de gélatine dans la cavité des rouleaux de confection d'une machine d'encapsulation à matrice rotative ; l'injection par un injecteur prévu sur la fermeture de la cavité, d'une quantité d'injectat médicamenteux correspondant à 1 à 60 % en poids, de préférence de 10 % à 50 % en poids, de la quantité de mélange de gélatine positionné dans la cavité, ledit injectat médicamenteux comprenant
20 à 60 % en poids d'un excipient adjuvant choisi dans le groupe constitué d'alcools polyhydroxylés et de polyéther-alcools,
0 à 50 % en poids d'éthanol,
10 à 50 % en poids d'eau,
0 à 55 % en poids de gélatine,
la quantité requise, en poids, du médicament utilisé dans la thérapie de narcomanie,
- le découpage et le retrait de la composition pharmaceutique dans une matrice de capsule molle uniforme ainsi formée, à partir de la machine à matrice rotative, et
- le séchage de la composition pharmaceutique dans la matrice de capsule molle uniforme.

14. Procédé selon la revendication 13, dans lequel l'injectat médicamenteux comprend 25 % à 42 % en poids d'un excipient adjuvant choisi dans le groupe constitué d'alcools polyhydroxylés et de polyéther-alcools, 0 % à 30 % en poids d'éthanol, 10 % à 45 % en poids d'eau, 10 % à 45 % en poids de gélatine, la quantité requise, en poids, du médicament utilisé dans la thérapie de narcomanie.
